# EUROPEAN PATENT SPECIFICATION

(11) **EP 2 621 890 B1**
(45) Date of publication and mention of the grant of the patent: **22.07.2015**
(21) Application number: 11764060.7
(22) Date of filing: 21.09.2011
(51) Int. Cl.: C07C 211/63, C07C 215/40

(54) **QUATERNARY AMMONIUM POLYHYDROXYL COMPOUNDS AND THEIR USE IN PERSONAL CARE COMPOSITIONS**
QUARTÄRE AMMONIUM-POLYHYDROXYL-VERBINDUNGEN UND IHRE VERWENDUNG FÜR KÖRPERPFLEGEZUSAMMENSETZUNGEN
COMPOSÉS POLYHYDROXYLÉS D'AMMONIUM QUATERNAIRE ET LEUR UTILISATION DANS DES COMPOSITIONS DE SOINS PERSONNELS

(30) Priority: 30.09.2010 US 388226 P
(43) Date of publication of application: 07.08.2013
(73) Proprietor: Dow Global Technologies LLC, Midland, MI 48674 (US)
(72) Inventor: DEAVENPORT, Joseph L., Lake Jackson TX 77566 (US); BREHM, Nicole A., Lake Jackson TX 77566 (US)
(74) Representative: Houghton, Mark Phillip
(86) International application number: PCT/US2011/052506
(87) International publication number: WO 2012/050769

(56) References cited:
- EP-A1- 2 189 152
- DE-C1- 19 517 048
- US-A- 4 083 872
- US-A1- 2008 299 237

## Description

### Field

The present invention relates to novel monocationic polyhydroxyl compounds and their uses in personal care compositions.

### Background

Polyhydroxyl compounds, or polyols, have a number of uses, from raw materials used in the manufacture of urethane foams to humectants for personal care products like shaving foams, lotions, and shampoos.

Quaternary ammonium compounds are also useful in a number of applications, such as for disinfectants, surfactants, fabric softeners, and conditioners in shampoos.

Despite the number of available conventional compounds, there is a strong need for novel compounds with properties to differentiate performance or offer synergistic effects in areas of interest, particularly in personal care compositions.

US 4083872 and DE 19512048 describe known quaternary ammonium polyhydroxy compounds.

### Detailed Description

In one embodiment, the present invention provides compounds, including salts, of the Formula (I): 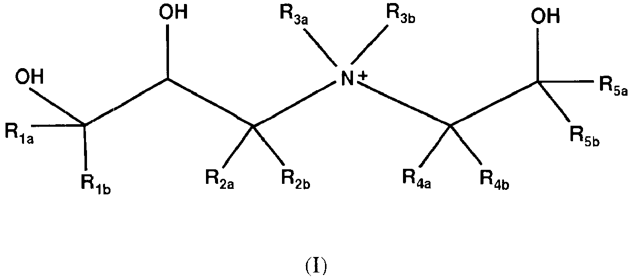 wherein:
A₁ₐ, R_{1b}, R₂ₐ, R_{2b}, R₄ₐ, R_{4b}, R₅ₐ, and R_{5b}, are, independently, H, optionally substituted
C₁-C₆ alkyl, or R₄ₐ and R_{4b} may cooperate to form a cycloalkyl; is -CH₃, and
R_{3b} is R₃ₐ -CH₂CH(OH)CH₂OH.

The term "optionally substituted" as used herein means that the groups in question are either unsubstituted or substituted with one or more groups, radicals or moieties, selected from halogen, hydroxy, amino or carboxy. When the groups in question are substituted with more than one substituent, the substituents may be the same or different. In one embodiment, the optional substituent is selected to produce a cosmetically acceptable compound. "Cosmetically acceptable" refers to ingredients typically used in personal care compositions, and is intended to underscore that materials that are toxic, irritating, or unpleasant smelling when present in the amounts typically found in personal care compositions are not contemplated as part of the present invention. In one embodiment, the optional substituent is one or more hydroxy groups.

"Alkyl" means a saturated monovalent linear or branched aliphatic hydrocarbon radical. Representative examples include, but are not limited to methyl, ethyl, propyl, isopropyl, butyl, isobutyl, tert-butyl, sec-butyl, pentyl, isopentyl, neopentyl, hexyl, isohexyl, and the like.

The term "cycloalkyl" denotes a saturated monocyclic or bicyclic cycloalkyl group. Examples of cycloalkyl groups include cyclopropyl, cyclobutyl, cyclopentyl, cyclohexyl, cycloheptyl, cyclooctyl, and the like. In one embodiment, the cycloalkyl is cyclohexyl or cyclopentyl.

Salts means that a counter-ion is present, preferably halogen, more preferably Cl⁻. In one embodiment, R₁ₐ and R_{1b} are each H.

In one embodiment, R₂ₐ and R_{2b} are each H.

In one embodiment, R₃ₐ is -CH₃ and R_{3b} is -CH₂CH(OH)CH₂OH.

In one embodiment, R₄ₐ is -CH₂OH.

In one embodiment, R_{4b} is -CH₂CH₃.

In one embodiment, R₄ₐ and R_{4b} cooperate to form a cyclohexyl group.

Other embodiements are contemplated, for example, the reaction product of 3-chloro-1,2-propanediol and monomethylamine .

In one embodiment, the present invention provides methods for providing humectancy in a personal care composition, comprising including the compound of Formula I into the personal care composition. The ingredients used, and their proportions and manner of addition, are familiar to those versed in conventional personal care compositions, including, optionally, cosmetically acceptable emollients, moisturizers, conditioners, oils, sunscreens, surfactants, emulsifiers, preservatives, rheology modifiers, colorants, preservatives, pH adjustors, propellants, reducing agents, fragrances, foaming or de-foaming agents, tanning agents, depilatory agents, astringents, antiseptics, deodorants, antiperspirants, insect repellants, bleachers, lighteners, anti-dandruff agents, adhesives, polishes, strengtheners, fillers, barrier materials, or biocides.

In one embodiment, the present invention provides hair care compositions containing the compound of Formula I.

In one embodiment, the present invention provides skin care compositions containing the compound of Formula I.

### Examples

### Comparative Example 1

### Reaction of 3-chloro-1,2-propanediol and dimethylaminobutanol to afford 2-(2,3-dihydroxypropyldimethylammonium)-1-butanol chloride

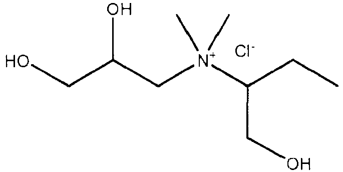

A 100 mL round bottom, jacketed, flask was equipped with a magnetic stirbar and placed on a stir-plate. The temperature was set to 20°C and monitored with a thermocouple. Dimethylaminobutanol, 23.56g (0.2 mol), was added to the reactor flask. Next, 21.86g (0.2 mol) of 3-chloro-1,2-propanediol was added dropwise over about 10 minutes to prevent too exothermic a reaction, i.e., the temperature was maintained at less than 30°C. The mixture was allowed to react for about 1 hour at 20°C, and then increased to 40°C.

3g of 49.6% NaOH solution was added to increase the pH to about 11. The mixture reacted for an additional 3 hours forming a thick white grey suspension. The temperature was increased to 50°C and roughly 20g of water was added to decrease the viscosity of the mixture. The mixture was then allowed to react for about 12 hours, NMR confirmed reaction completion. The pH was adjusted from about 12 to roughly neutral using concentrated HCl.

¹³C NMR spectra acquired from a Bruker 300MHz spectrometer (samples prepared as ∼30 wt% in D₂O) confirmed the title compound: DEPT NMR (250 MHz, D2O) 10.5, (17.6, 17.7), (49.7, 50.3, 50.5), 56.7, 63.7, (65.4, 65.6, 66.1), 68.1, (76.1, 76.4).

### Comparative Example 2

### Reaction of 3-chloro-1,2-propanediol and 3-Dimethylamino-4-hydroxyoctane to afford 3-(2,3-dihydroxypropyldimethylammonium)-4-hydroxyoctane chloride

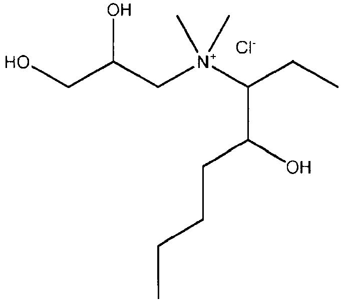

The title compound is prepared substantially according to the protocol of Example 1, except that the conditions and amounts of reactants may vary, typically a 1:1 mol ratio with a slight excess of 3-chloro-1,2-propanediol, but which factors are well within the skill of one ordinarily skilled in the art.

### Comparative Example 3

### Reaction of 3-chloro-1,2-propanediol and 2-Dimethylamino-1,3-propanediol to afford 2-(2,3-dihydroxypropyldimethylammonium)-1,3-propanediol chloride

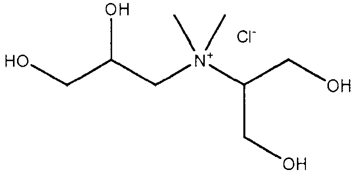

The title compound is prepared substantially according to the protocol of Example 1, except that the conditions and amounts of reactants may vary, typically a 1:1 mol ratio with a slight excess of 3-chloro-1,2-propanediol, but which factors are well within the skill of one ordinarily skilled in the art.

### Comparative Example 4

### Redaction of 3-chloro-1,2-propanediol and 2-Dimethylamino-2-methyl-1,3-propanediol to afford 2-(2,3-dihydroxypropyldimethylammonium)-2-methyl-1,3-propanediol chloride

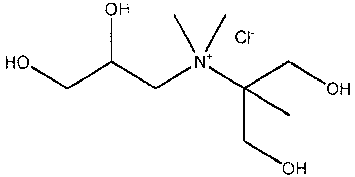

The title compound is prepared substantially according to the protocol of Example 1, except that the conditions and amounts of reactants may vary, typically a 1:1 mol ratio with a slight excess of 3-chloro-1,2-propanediol, but which factors are well within the skill of one ordinarily skilled in the art.

### Comparative Example 5

### Redaction of 3-chloro-1,2-propanediol and N,N-Dimethyl-2-amino-2-methylbutan-1-ol to afford 2-(2,3-dihydroxypropyldimethylammoniun)-2-methyl-1-butanol chloride

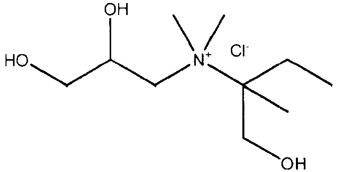

The title compound is prepared substantially according to the protocol of Example 1, except that the conditions and amounts of reactants may vary, typically a 1:1 mol ratio with a slight excess of 3-chloro-1,2-propanediol, but which factors are well within the skill of one ordinarily skilled in the art.

### Comparative Example 6

### Reaction of 3-chloro-1,2-(Dimethylamino)-2-ethyl-1,3-propanediol to afford 2-(2,3-dihydroxypropyldimethylammonium)-2-ethyl-1,3-propanediol chlorine

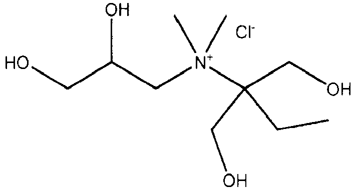

The title compound is prepared substantially according to the protocol of Example 1, except that the conditions and amounts of reactants may vary, typically a 1:1 mol ratio with a slight excess of 3-chloro- 1,2-propanediol, but which factors are well within the skill of one ordinarily skilled in the art.

### Comparative Example 7

### Reaction of 3-chloro-1,2-propanediol and 2-(Dimethylamino)-2-(hydroxymethyl)-1,3-propanediol to afford 2-(2,3-dihydroxypropyldimethylammonium)-2-hydroxymethyl-1,3-propanediol chloride

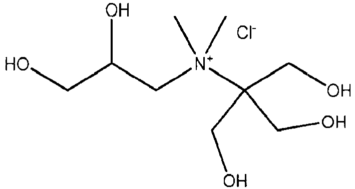

The title compound is prepared substantially according to the protocol of Example 1, except that the conditions and amounts of reactants may vary, typically a 1:1 mol ratio with a slight excess of 3-chloro-1,2-propanediol, but which factors are well within the skill of one ordinarily skilled in the art.

### Comparative Examples 8

### Reaction of 3-chloro-1,2-propanediol and 2-Dimethylamino-2-methylpropanol to afford 2-(2,3-dihydroxypropyldimethylammonium)-2-methylpropanol

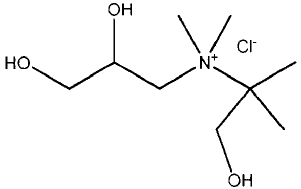

The title compound is prepared substantially according to the protocol of Examples 1, except that the conditions and amounts of reactants may vary, typically a 1:1 mol ratio with a slight excess of 3-chloro-1,2-propanediol, but which factors are well within the skill of one ordinarily skilled in the art.

### Comparative Example 9

### Reaction of 3-chloro-1,2-propanediol and VANTEX®-T amine to afford 2,3-dihydroxypropyl(di)hydroxyethylbutylammonium chloride

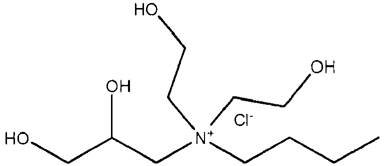

The title compound is prepared substantially according to the protocol of Example 1, except that the conditions and amounts of reactants may vary, typically a 1:1 mol ratio with a slight excess of 3-chloro-1,2-propanediol, hut which factors are well within the skill of one ordinarily skilled in the art.

### Comparative Example 10

### Reaction of 3-chloro-1,2-propanediol and 1-(Dimethylamino)cyclohexanemethanol to afford 2,3-dihydroxypropyldimethylcyclohexanemethanolammonium chloride

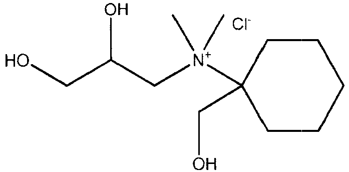

The title compound is prepared substantially according to the protocol of Example 1, except that the conditions and amounts of reactants may vary, typically a 1:1 mol ratio with a slight excess of 3-chloro-1,2-propanediol, but which factors are well within the skill of one ordinarily skilled in the art.

### Comparative Example 11

### Reaction of 3-chloro-1,2-propanediol and 1-Dimethylamino-2-propanol to afford 3-(2,3-dihydroxypropyldimethylammonium)-2-propanol chloride

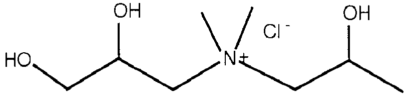

The title compound was prepared substantially according to the protocol of Example 1, except that 19.97 g (0.19 mol) of 1-Dimethylamino-2-propanol was reacted with 20.79g (0.19 mol) of 3-chloro-1,2-propanediol; 0.53 g 49.6% NaOH solution was added to bring the pH above 12; the mixture was allowed to react for 5 hours at 20°C; and then the pH was increased to 12.3 (using 0.48g of 49.6% NaOH solution) and the temperature was increased to 40°C, and after about 1.5 hours, the temperature was increased to 60°C.

¹³C NMR spectra acquired from a Bruker 300MHz spectrometer (samples prepared as ∼30 wt% in D₂O) confirmed the title compound: DEPT NMR (250 MHz, D2O) 21.3, (52.5, 52.6, 52.9), (62.1, 62.2), 63.7, (66.1, 66.2), (66.9, 67.3), (70.1, 70.5).

### Example 12

### Reaction of 3-chloro-1,2-propanediol and monomethylamine to afford tris(2,3-dihydroxypropyl)methylammonium chloride

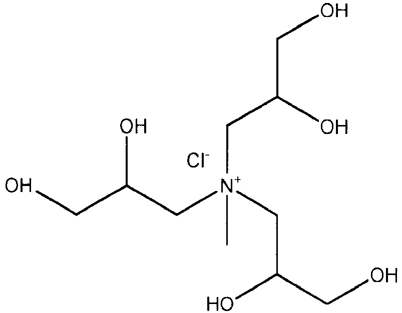

The title compound was prepared substantially according to the protocol of Example 1, except that 9.24 moles 3-chloro-1,2-propanediol was reacted with 2.96 moles monomethylamine; sufficient 49.6% NaOH solution to achieve a pH of about 10.5 to about 11 was added; the reaction was maintained at 25°C for an hour, then increased to about 50°C for about 2.5 hours; additional 49.6% NaOH solution to achieve a pH of about 10.5, then the reaction continued for 16 hours. Excess 3-chloro-1,2-propanediol was scavenged with trimethylamine.

¹³C NMR spectra acquired from a Bruker 300MHz spectrometer (samples prepared as ∼30 wt% in D₂O confirmed the title compound: DEPT NMR (250 MHz, D2O) 51.1, (63.8, 63.9), 65.7, (65.9, 66.0, 66.1).

### Example 13

Compounds prepared substantially according to Comparative Examples 1-11 and Example 12 are made and formulated into personal care compositions having otherwise conventional ingredients. The compositions are evaluated by trained panelists, with each panelist being asked to compare the inventive compositions to a conventional composition.

For hair care compositions, wet and dry feel preference and wet and dry combability is measured by asking the panelists to feel and comb two hair tresses of European virgin brown hair, commercially available from International Hair Importers and Products Inc. NY (USA), one hair tress treated with an inventive composition, the other hair tress treated with a conventional composition. Each panelist is asked to compare the tresses and state which tress is smoother to comb / feel. The answer "same" is not allowed. The reported number is the percent of panelists preferring one over the other.

For skin care compositions, panelists apply a sample (one inventive composition, one conventional composition) to a designated area on their right or left forearm. Initially, each sample is evaluated for ease of application, play time, evenness of deposit, coverage, speed of adsorbtion, shine, matte, skin moistness, heaviness, amount of grease, amount of tack, quickness of drying, overall skin feel, and overall appearance. After a designated time, each sample is again evaluated, this time for coverage, evenness of coverage, shine, matte, skin moistness, heaviness, and overall appearance.

## Claims

1. A compound, including salts, of the Formula (I): 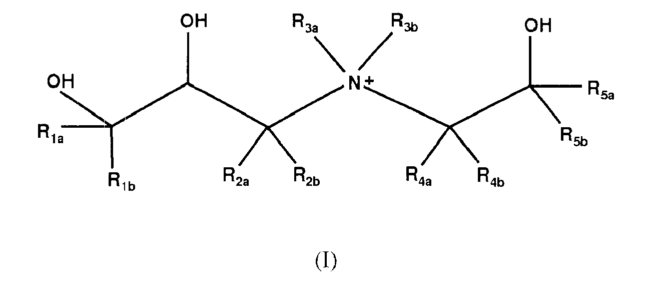 wherein:
R₁ₐ, R_{1b}, R₂ₐ, R_{2b}, R₄ₐ, R_{4b}, R₅ₐ, and R_{5b}, are, independently, H, optionally substituted C1-C6 alkyl, or R₄ₐ and R_{4b} may cooperate to form a cycloalkyl; and
R₃ₐ is -CH₃, and R_{3b} is -CH₂CH(OH)CH₂OH.

2. The compound of Claim 1, wherein R₄ₐ is -CH₂OH.

3. The compound of Claim 1, wherein R_{4b} is -CH₂CH₃.

4. The compound of Claim 1, wherein R₄ₐ and R_{4b} cooperate to form a cyclohexyl group.

5. A method for providing humectancy in a personal care composition, comprising including the compound of claim 1 into the personal care composition.

6. A hair care composition containing the compound of claim 1.

7. A skin care composition containing the compound of claim 1.

## Patentansprüche

1. Eine Verbindung, einschließlich Salzen, der Formel (I): 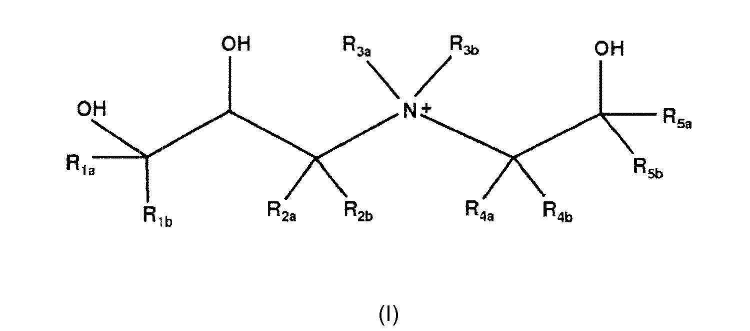 wobei:
R₁ₐ, R_{1b}, R₂ₐ, R_{2b}, R₄ₐ, R_{4b}, R₅ₐ und R_{5b} unabhängig H, optional substituiertes C1-C6-Alkyl sind oder R₄ₐ und R_{4b} kooperieren können, um ein Cycloalkyl zu bilden; und
R₃ₐ -CH₃ ist und R_{3b} -CH₂CH(OH)CH₂O ist.

2. Verbindung gemäß Anspruch 1, wobei R₄ₐ -CH₂OH ist.

3. Verbindung gemäß Anspruch 1, wobei R_{4b} -CH₂CH₃ ist.

4. Verbindung gemäß Anspruch 1, wobei R₄ₐ und R_{4b} kooperieren, um eine Cyclohexylgruppe zu bilden.

5. Ein Verfahren zum Bereitstellen einer Feuchthalteeigenschaft in einer Körperpflegezusammensetzung, beinhaltend das Einschließen der Verbindung gemäß Anspruch 1 in die Körperpflegezusammensetzung.

6. Eine Haarpflegezusammensetzung, enthaltend die Verbindung gemäß Anspruch 1.

7. Eine Hautpflegezusammensetzung, enthaltend die Verbindung gemäß Anspruch 1.

## Revendications

1. Un composé, incluant des sels, de la formule (I) : 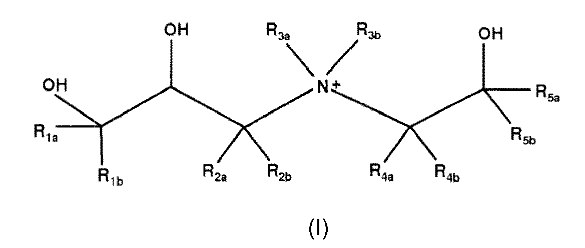 où :
R₁ₐ, R_{1b}, R₂ₐ, R_{2b}, R₄ₐ, R_{4b}, R₅ₐ, et R_{5b}, sont, indépendamment, H, un alkyle en C1 à C6 facultativement substitué, ou R₄ₐ et R_{4b} peuvent coopérer pour former un cycloalkyle ; et
R₃ₐ est-CH₃, et R_{3b} est-CH₂CH(OH)CH₂OH.

2. Le composé de la revendication 1, où R₄ₐ est -CH₂OH.

3. Le composé de la revendication 1, où R_{4b} est -CH₂CH₃.

4. Le composé de la revendication 1, où R₄ₐ et R_{4b} coopèrent pour former un groupe cyclohexyle.

5. Une méthode pour fournir une propriété humectante dans une composition de soins personnels, comprenant le fait d'inclure le composé de la revendication 1 dans la composition de soins personnels.

6. Une composition de soins pour les cheveux contenant le composé de la revendication 1.

7. Une composition de soins pour la peau contenant le composé de la revendication 1.
